# EUROPEAN PATENT SPECIFICATION

(11) **EP 3 681 421 B1**
(45) Date of publication and mention of the grant of the patent: **20.10.2021**
(21) Application number: 18769459.1
(22) Date of filing: 10.09.2018
(51) Int. Cl.: A61B 17/68, A61B 17/88

(54) **ADJUSTABLE BONE IMPLANTS**
VERSTELLBARE KNOCHENIMPLANTATE
IMPLANTS OSSEUX RÉGLABLES

(30) Priority: 11.09.2017 GB 201714574
(43) Date of publication of application: 22.07.2020
(73) Proprietor: Sikander, Murtuza, Northwood, London HA6 3NE (GB)
(72) Inventor: Sikander, Murtuza, Northwood, London HA6 3NE (GB)
(74) Representative: HGF
(86) International application number: PCT/GB2018/052558
(87) International publication number: WO 2019/048884

(56) References cited:
- WO-A1-2014/207284
- US-A1- 2012 165 879

## Description

This invention relates to adjustable bone implants. In particular, but not exclusively, the invention relates to bone implants that enable a bone flap to be secured in a raised position following decompressive hemi-craniectomy surgery and to be lowered back to a normal anatomical position after a period of time.

### BACKGROUND

Following a traumatic brain injury or in stroke patients, the brain tissue may swell, causing an increase in intracranial pressure. In severe cases, this increase in intracranial pressure can restrict blood flow to areas of the brain and cause subsequent damage to originally undamaged brain tissue. The increased intracranial pressure can cause the brain to deform and in extreme cases, a shift in the midline of the brain by 2 cm in a closed cranial vault can be fatal. To reduce the intracranial pressure, a decompressive hemi-craniectomy can be performed. This involves incising the scalp over the side of the injured brain and removing a large section of the skull, typically about the size of the patient's open hand, and referred to as a bone flap, which allows the brain tissue to swell and expand into the surgically created defect. Studies have shown that a bone flap that is at least 12 cm in length and of adequate width is adequate to increase the volume sufficiently to prevent midline shift. The scalp is then re-sutured over the swollen brain which provides minimal, soft-tissue protection to the underlying brain.

The swelling typically takes a few weeks to subside. Meanwhile, it is important to preserve the sterility of the bone flap and this may be achieved by temporarily implanting it into the fatty layer of the patient's abdominal wall or in a sterile bone bank. After the swelling has subsided, the patient undergoes a second surgical intervention called a cranioplasty to reconstruct the skull defect using either the preserved bone flap or a biocompatible synthetic implant.

Once a patient is ready to undergo further surgery to receive the bone flap or cranioplasty implant, a surgeon will need to reopen the scalp and expose the skull and brain tissue to the environment so that the bone flap can be reattached and secured in place with bone screws or clamps.

During the period between the first, decompressive hemi-craniectomy, and second, cranioplasty, surgical procedures, there are many risks to the patient. Creation of a large cranial defect exposes the patient to the risk of further trauma to the brain. Most patients undergoing a decompressive hemi-craniectomy may not be well enough to undergo the subsequent cranioplasty for a period longer than the two weeks it takes the brain swelling to subside and often cranioplasty is delayed for weeks to months following the decompressive hemi-craniectomy. Delayed reconstruction of the skull defect increases the risk of infection associated with the preserved bone flap and has surgical risks associated with cranial fluid leaks due to scarring between the brain tissue lining and the inner scalp. There is also an increased risk of wound breakdown as surgery is carried out through scar tissue. Additionally, preserving the bone flap in the abdominal wall requires a further incision in the abdominal area which has further associated risks and there may be absorption of bone by fatty tissue if left in place for prolonged periods. Synthetic cranioplasty implants are bio-mechanically inferior to bone and are expensive, carrying their own specific complications related to infection and cosmesis. Moreover, although the effects of prolonged direct atmospheric pressure on the brain are not completely understood, a syndrome: "syndrome of the trephined" or "sunken flap syndrome" has been described in the medical literature relating to worsening of neurological functioning after subsidence of brain swelling with decompressive hemi-craniectomy.

Rather than completely removing the bone flap for the intervening period between the surgical procedures, surgical techniques of placing a 'free floating' bone flap or a 'hinged' bone flap over the brain have been used in the past. These techniques leave the bone flap unsecured over the swollen brain or hinged on one side of the skull. These approaches expose the brain to pressure from the atmosphere and from the weight of the overlying bone flap. These techniques may not effectively allow for decompression of the swollen brain and also run the risk of the free edge of the bone flap being driven into the brain. These techniques may also have a poor cosmetic result. For these reasons, the techniques described above are not frequently used.

WO 2014/207284 A1 discloses a bone implant according to the preamble of claim 1.

It is an object of the invention to address at least some of these problems with the known surgical techniques associated with decompressive hemi-craniectomy procedures.

### BRIEF SUMMARY OF THE DISCLOSURE

The present invention provides an adjustable bone implant for expansile cranioplasty surgery according to claim 1. The implant comprises a body configured to be secured to a first bone surface; an anchoring member configured to be secured to a second bone surface held at a raised position offset from the first bone surface, and a spacing member connecting the anchoring member to the body in an adjustable manner, to selectively space the anchoring member from the body. The second bone surface comprises an upper surface of a bone flap, and the first bone surface comprises an upper surface of the remainder of the skull. The spacing member is located subcutaneously in use and is configured to receive an input via a transcutaneous transmission from an external tool. The spacing member is configured to lower the bone flap from a raised position upon receipt of the input and secure the bone flap at the lowered position. The lowering movement is perpendicular to the first and second bone surfaces. The external tool comprises a magnetic head and the spacing member comprises a magnetic portion, and the magnetic head and the magnetic portion are magnetically coupleable transcutaneously, whereby rotation of the external tool causes a rotation of the spacing member for adjustment thereof.

Advantageously, this allows the bone flap to be held securely at a set distance relative to the remainder of the skull for a period post implantation of the bone implant, for example to allow a healing process to occur. Then, the bone flap can be lowered back towards the remainder of the skull without a further invasive surgical intervention, due to the transcutaneous actuation of the spacing member. The implant has particular benefits when used for expansile cranioplasty surgery, because it enables the bone flap to be held in a secure, raised position for a period post-surgery while the swelling reduces, providing a safe, sterile way of protecting the healing brain tissue, and yet which also enables the bone flap to be lowered back to its natural position without a further surgical intervention by operation of the spacing member transcutaneously.

Where the adjustable bone implant has secured the bone flap in a lowered position, the first bone surface is typically aligned with the second bone surface and the first and second bone surfaces form a substantially contiguous surface. It is expected that the final position of the bone surfaces relative to one another would be aligned, so that the bone surfaces may heal into a single contiguous bone surface.

The body may comprise a hollow interior and the spacing member may be housed at least partially within the hollow interior. The body may comprise a sealed bottom. Thus, bodily fluids would be kept out of the body of the implant, away from the spacing member, thereby ensuring it can operate even after a period of time post implantation.

The spacing member may comprise a first member with a screw thread, matingly engaged with a corresponding screw thread on an inside surface of the body. Accordingly, the input may comprise rotation of the first member in a first direction, which causes an axial translation of the first member relative to the body to lower the bone flap from the raised position towards the lowered position, the axis of translation being perpendicular to the first and second bone surfaces. In an alternative embodiment, the spacing member may comprise an inner member with a screw thread matingly engaged with a corresponding screw thread on an outer member, which in turn has a further screw thread matingly engaged with a corresponding screw thread on an inside surface of the body. Accordingly, the input may comprise rotation of the inner member in a first direction, which causes an axial translation of the inner member relative to the outer member and in turn causes an axial translation of the outer member relative to the body to lower the bone flap from the raised position towards the lowered position, the axis of translation being perpendicular to the first and second bone surfaces. The screw thread on the first member may be either internal or external, with the corresponding screw thread of the second member respectively being either matingly external or internal. The first member is thus telescopically received in or over the second member, which in turn is telescopically received in or over the body, thereby forming a multiply telescopic arrangement. Such a telescopic arrangement may be beneficial to provide an increased range of adjustability for the spacing member for a given depth of the implant.

The magnetic portion may be made from any of an aluminium alloy, stainless steel or neodymium. The magnetic head may comprise one or more of either or both of: electromagnets or permanent magnets.

The body or anchoring member may be secured by at least two bone screws.

The spacing member may be extendable and retractable by up to 40mm to respectively raise or lower the bone flap relative to the remainder of the skull by a corresponding amount.

At least one of the spacing member, the anchoring member and the body may be made from titanium alloy or stainless steel.

The spacing member may have a recess configured to receive a head of the external tool and the recess may be accessed by a transcutaneous incision. The incision may be a small incision into the scalp located above the spacing member. By way of example, the external tool head may comprise any of a screwdriver head, a socket wrench head or an Allen key head. These mechanical interfaces may be provided in addition to the magnetic head. As such, a mechanical operation of the spacing member may be achieved in the event of difficulties in operation via the magnetic coupling alone.

The spacing member may be connected to the anchoring member by means of an aperture through the anchoring member. Alternatively, the spacing member may comprise a hollow cylindrical shroud member, the spacing member being connected to the anchoring member by means of an underside of a top of the anchoring member resting on top of the spacing member.

### BRIEF DESCRIPTION OF THE DRAWINGS

Embodiments of the invention are further described hereinafter with reference to the accompanying drawings, in which:
Figure 1 is an illustration of a patient with a bone flap held in a raised position above the remainder of their skull by three adjustable bone implants;
Figure 2 is an illustration of an adjustable bone implant according to one embodiment;
Figure 3 is an illustration of a bone flap held in a raised position by an adjustable bone implant according to one embodiment and allowing the swollen brain tissue to expand; and
Figure 4 is an illustration of a bone flap held in a lowered position by an adjustable bone implant according to one embodiment after the brain swelling has subsided.

### DETAILED DESCRIPTION

The present invention provides an adjustable bone implant configured to hold a bone flap securely in a raised position in a decompressive hemi-craniectomy procedure and for a period whilst brain swelling subsides and, once the swelling has subsided and after receiving an input via a transcutaneous transmission from an external tool, to move the bone flap from the raised position to a lowered position. This lowered position may typically bring the bone flap into alignment with the remainder of the skull, in a normal anatomical position. The implant comprises a body, an anchoring member and a spacing member to connect the anchoring member to the body in an adjustable manner, where at least the spacing member is located subcutaneously.

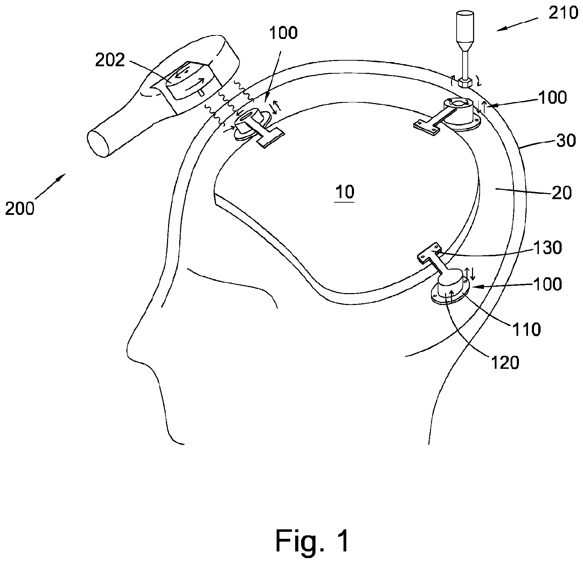

Figure 1 is an illustration of a patient with a bone flap 10 held in a raised position above the remainder of their skull 20 by three adjustable bone implants 100. The bone flap 10 may be held by three adjustable bone implants 100 spaced around the periphery of the bone flap 10, as shown, thereby creating a three-point fixation system which provides stability to the bone flap 10. It will be understood that in some circumstances either more than or fewer than three adjustable bone implants 100 could be used instead.

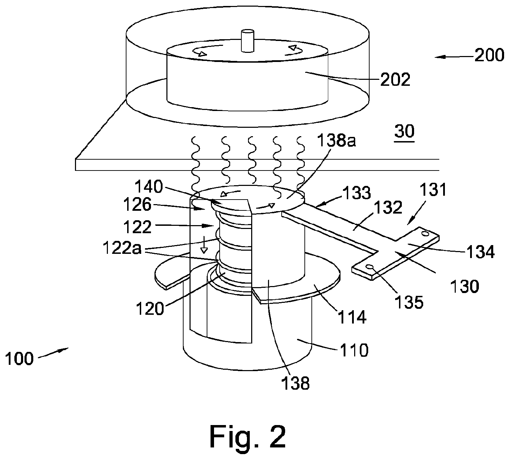

Each adjustable bone implant 100 comprises a body 110, an anchoring member 130 and a spacing member 120 to connect the anchoring member 130 to the body 110 in an adjustable manner to selectively space the anchoring member 130 from the body 110, as best shown in Figures 2 to 4.

The body 110 is for implantation into a hole 170 that has been drilled in the skull 20 at a location adjacent the periphery of the bone flap 10, as described more fully below. The body 110 comprises a generally cylindrical structure with a hollow, threaded interior surface 112 secured within the thickness of the skull bone 20. The body 110 is configured to receive the spacing member 120 within the hollow portion thereof. The body 110 has a rim 114 projecting radially outward of an upper edge, configured to receive a plurality of bone screws 160. The rim 114 includes a number of holes to receive respective bone screws 160 so that the body 110 can be secured to the remainder of the skull 20 about the upper periphery of the hole 170. It will be understood that the holes in the rim are optional and that self-tapping bone screws might be used to pierce and pass through a rim that is not provided with such holes. Self-tapping screws may be preferable to pre-drilled holes, as this gives the surgeon the flexibility of refining the location and method of fixation of the implant 100 during the surgical procedure. The body 110 has a sealed bottom portion 150 to prevent any fluids or brain tissue 40 from entering the bone implant 100 through the underside of the hole 170. If the hole 170 is a blind hole, then the bottom of the insert body 110 could be open because there would be no fluid path into the interior of the body past the bone at the bottom of the blind hole.

Whereas the body 110 is shown being secured to the skull bone 20 by bone screws 160, clamps or other such bone fixation devices are equally suitable. Similarly, while it is expedient to use two screws 160 to provide a stable connection between the body 110 and the skull 20, greater or fewer than two screws, including no screws, may be used. By using two bone screws 160, this protects against implant loosening if one of the screws 160 were to fail. Where no screws are used, the rim 114 may be omitted and the body 110 may be secured in position by friction between an outer surface of the body 110 and the skull 20 caused by an interference fit and/or by surface features on the body 110, such as abrasions, protrusions, barbs, etc., that can engage with the bone material and provide enhanced mechanical fixation compared to a smooth surface. The outer surface of the body 110 may also be provided with features to encourage bone in-growth.

The anchoring member 130 comprises a T-shaped, substantially flat structure comprising a central span 132 extending in a longitudinal direction between a first end 131 and a second end 133, and a securing portion 134 extending at the first end 131 of the central span in a direction substantially perpendicular to the longitudinal direction thereof and which secures the anchoring member 130 to the bone flap 10. In the illustrated embodiment, the central span 132 and the securing portion 134 are integrally formed, but they could be separate pieces that are connected together. As shown, the securing portion 134 has two pre-drilled holes 135 to receive two bone screws 160 which are used to secure the anchoring member 130 to the bone flap 10. The securing portion 134 may take other shapes and indeed may be omitted if the central span 132 is configured to receive bone screws for fixation to the bone flap 10. As with the rim of the body 110, it is not necessary to provide pre-drilled holes to receive the bone screws 160, which could instead form their own holes through the anchoring portion as they are inserted, if the materials are suitable.

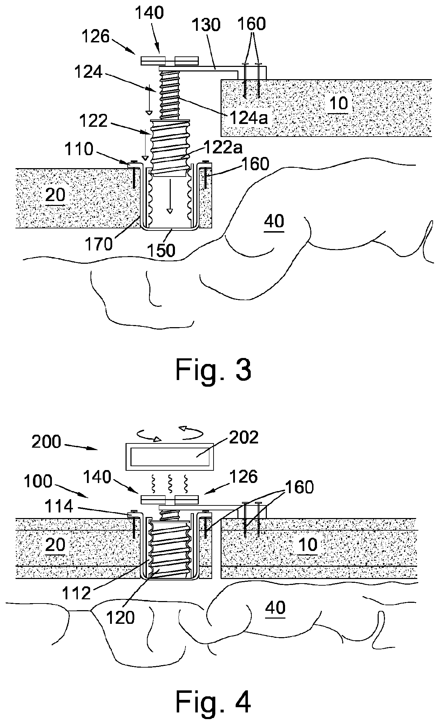

The second end 133 of the central span 132 is connected to the spacing member 120 so as to be raised and lowered with movement of the spacing member. In one embodiment, as shown in Figures 3 and 4, there is an aperture through the second end 133, which is received on an upper end of the spacing member 120, as described in greater detail below. In another embodiment, as shown in Figure 2, there is a hollow cylindrical shroud 138 at the second end. The shroud has a circular top 138a, the underside of which rests on the top of the spacing member 120, as described in greater detail below. As the anchoring member 130 is connected to the bone flap 10, translation of the anchoring member 130 by means of the spacing member 120 results in a similar translation of the bone flap 10. While two screws are preferable for connecting the anchoring member 130 to the bone flap 10, as they stop the anchoring member 130 rotating relative to the bone flap 10, greater or fewer bone screws 160 may be used instead. While bone screws 160 have been described as the preferred method of fixation, clamps or other such bone fixation devices may be equally suited.

The spacing member 120 comprises an outer member 122 and an inner member 124. The outer member 122 has a threaded exterior surface 122a for threaded engagement with the correspondingly threaded interior surface 112 of the body 110, such that rotation of the outer member 122 in a first direction (typically anti-clockwise) results in an axial translation of the outer member to project out of the body. A stop (not shown) may be provided to prevent the outer member 122 from inadvertently being removed from the body 110. Correspondingly, rotation in the opposite direction results in the outer member 122 axially translating back into the hollow interior of the body 110. The inner member 124 also has a threaded exterior surface 124a, for engagement with a correspondingly threaded bore (not shown) within the outer member 122, whereby rotation of the inner member 124 within the outer member 122 results in an axial translation of the inner member 124 relative to the outer member 122. A head portion 126 of the inner member 124 includes input-receiving means to receive an input from an external tool 200, 210 for adjustment of the spacing member 120.

The input-receiving means is in the form of a magnetic portion 140 on the head portion 126, which is magnetically coupleable with a corresponding magnetic head 202 on the external tool 200. The interacting magnetic fields are of sufficient strength that the magnetic portion 140 and the magnetic head 202 can be coupled transcutaneously such that rotation of the magnetic head 202 externally of the patient's scalp results in a corresponding rotation of the magnetic portion 140 that is located subcutaneously and hence in the adjustment of the offset spacing of the bone flap 10 relative to the remainder of the skull 20. The magnetic head 202 may comprise an arrangement of electromagnets and/or permanent magnets. The magnetic portion 140 may be made from any suitable magnetic material, such as a magnetic alloy, aluminium alloy, stainless steel or neodymium.

The implant 100 may be supplied and implanted with the spacing member in an extended (raised) configuration, since the initial use of the implant will be to space the bone flap 10 from the remainder of the skull 20. It is envisaged that the adjustment of the spacing member 120 could, in some embodiments, be one-way only: to lower the anchoring member 130 towards the body 110.

In the lowering direction, as the tool 200 is turned in a clockwise direction, the magnetic coupling between the head 202 and the magnetic portion 140 of the inner member 124 results in a clockwise rotation of the inner member 124 which, by way of the mating threaded coupling with the outer member 122, results in the inner member 124 translating axially into the core of the outer member 122. When the inner member 124 has reached a limit of insertion within the outer member 122, continued rotation of the tool 200 in the clockwise direction will cause the outer member 122 to likewise turn in the clockwise direction. By virtue of the engagement of the outer threaded surface 122a with the inner thread 112, the outer member 122, with the inner member 124 held within, translates axially into the hollow interior of the body 110. Thus, the anchoring member 130 may be lowered to a position in which the bone flap 10 is substantially contiguous with the remainder of the skull 20.

However, given that adjustment in either direction could be beneficial, for example to re-raise the bone flap 10, it is envisaged that the spacing member 120 would typically be adjustable in either direction: lowering or raising the anchoring member 130 relative to the body 110. The operation to raise the anchoring member 130 is the converse to its lowering: as the tool 200 is rotated anti-clockwise, first the inner member 124 is translated axially out of the outer member 122 until a limit of travel is reached, at which point continued rotation in the anti-clockwise direction would cause the outer member 122 then to extend out of the body 110.

The spacing member 120 effectively operates in a telescopic manner, where the inner and outer members 122, 124 of the spacing member 120 are each received within the hollow interior of the body 110 as the anchoring member 130 is lowered. In certain embodiments, the outer member 122 may be omitted, with the outer threaded surface 124a of the inner member 124 being matingly received in the internal thread 112 of the body 110. The threaded inner surface 112 may be considered as part of the spacing member 120. For a given range of adjustment, having the spacing member comprise two or more telescopically concentric members means that a lower profile body 110 can be used. This may be advantageous to keep the height of the implant 100 to a minimum.

The implants 100 would be implanted during a first decompressive hemi-craniectomy. During this surgical procedure the surgeon has made an incision into the scalp and pulled the scalp back to reveal the area of the skull which will become the bone flap 10. This would allow the surgeon to prepare the required implants 100 and the skull by drilling suitably sized holes 170 into the remaining skull 20 to accommodate the respective bodies 110 of the implants. Bone screws 160 would typically be used to secure the bodies 110 within the respective holes 170 and to secure the associated anchoring members 130 in position on the bone flap 10. The surgeon can set the implants 100 to the desired height so that the bone flap 10 can be secured in the raised position over the brain. Once the implants 100 are configured correctly and the bone flap 10 has been secured in the desired position, the scalp 30 can be pulled back over the defect site and sutured closed to seal the defect site with the bone flap 10 held in a raised position.

Once implanted, each bone implant 100 would be located subcutaneously and remain below the scalp 30. Preferably, the implant would be controlled remotely by the external tool 200. The body 110, spacing member 120 and anchoring member 130 may be made from any biocompatible material, such as carbon fibre, titanium, titanium alloy or stainless steel.

The term "transcutaneous transmission" used throughout the description should be taken to mean any transmission that passes through the skin, without damaging the skin. A transcutaneous transmission does not require physical contact with the spacing member 120 to cause rotation or translation of the spacing member 120. Such a transmission could also be regarded as a "contactless transmission".

The bone flap 10 can be lowered from a raised position to a lowered position after an input has been received by the implant. This lowered position may be at any level below the raised position. When the brain swelling has subsided, the bone flap 10 would be lowered to an anatomical position to provide a good cosmetic result without the need for a second surgery. As described above, the input is provided by an external tool 200 located outside the body, which is able to remotely provide the input to the implant 100 and cause the implant 100 to lower the bone flap 10. The use of inter-engaging screw threads means that continuously variable positioning can be achieved, with the bone flap 10 being held securely at any set height.

Figure 3 is an illustration of a bone flap 10 held in a raised position by an adjustable bone implant 100 and allowing the swollen brain tissue 40 to expand. As shown, the brain tissue 40 is swollen and protruding out of skull 20 and the implant 100 is in an open configuration with the bone flap 10 held in a raised position. In some cases, the brain tissue 40 will protrude out of the skull by 20-30 mm. To accommodate this, the implant 100 would be adjustable such that the bone flap 10 could be held in a raised position offset from the skull surface 20 by at least 20 mm, but preferably up to 40 mm to allow for adequate volume increase and prevent a fatal increase in intracranial pressure.

Figure 4 is an illustration of a bone flap 10 held in a lowered position by an adjustable bone implant 100 after the brain swelling has subsided. Once the brain tissue 40 is no longer swollen, the external tool 200 or 210 can be used to lower the bone flap 10 to the skull 20, such that the bone flap 10 and skull 20 form a substantially contiguous surface. This restores the bone flap 10 to its anatomical position and results in good cosmesis without the need for a second surgery. As shown, the implant 100 is in a closed configuration, where the spacing member 120 is substantially contained within the body 110 of the implant 100 and only the anchoring member 130 and a small portion of the spacing member 120 remains above the substantially contiguous surface formed by the bone flap 10 and the skull 20. However, obvious variations on the design of the connections between the spacing member 120 and the anchoring member 130 and the method of securing the anchoring member 130 to the bone flap 10 would result in none of the implant 100 remaining above the bone surface.

It may be necessary to access the implant 100 directly. In this case, an external tool 210 could be brought into direct mechanical contact with the implant 100 to adjust the spacing member 120 through an incision into the scalp 30. For example, the external tool 210 might be any of a screwdriver, a socket wrench or an Allen key, the spacing member 120 having an appropriate slot or recess to receive the head of such a tool for the transmission of torque therebetween. Even in this scenario, the surgical procedure to access and adjust the implant 100 would be preferable to existing approaches, as only small incisions at the location of each implant 100 would be required.

By securing the bone flap 10 in position above the brain in this way, the brain can be better protected than would be provided by the patient's scalp alone. This would be not just for the time the brain is swollen, but for the entire time the bone flap 10 is separated from the skull 20. Securing the bone flap 10 in this manner is preferable to existing methods of re-attaching the bone flap 10 by incising the scalp 30. By using a magnetic field to lower the bone flap 10 into position, there is no need for further incisions into the scalp 30, which reduces the risk of infection. A further advantage of the present invention is there is no need to separately preserve the bone flap 10 by freezing or storing within the patient's abdomen while they recover from the initial head injury. As the bone flap 10 is kept within the body it will be naturally preserved until it can be lowered back into the skull 20.

In summary the present invention provides a bone implant that is located subcutaneously which can secure a bone flap above the exposed brain while providing the brain room to expand into and reduce the intracranial pressure. Once brain swelling has subsided, the bone implant can be adjusted by an external tool to lower the bone flap from a raised position to a lowered position and align the bone flap with the rest of the skull. This eliminates the need for a second major surgical procedure and provides significantly better protection to the brain during the patient's recovery period.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The scope of the invention is defined by the appended claims.

## Claims

1. An adjustable bone implant (100) for expansile cranioplasty surgery, comprising:
a body (110) configured to be secured to a first bone surface;
an anchoring member (130) configured to be secured to a second bone surface held at a raised position offset from the first bone surface, wherein the second bone surface comprises an upper surface of a bone flap (10) and the first bone surface comprises an upper surface of the remainder of the skull (20); and
a spacing member (120) connecting the anchoring member to the body in an adjustable manner, to selectively space the anchoring member from the body,
wherein the spacing member is located subcutaneously in use,
wherein the spacing member is configured to receive an input via a transcutaneous transmission from an external tool (200), and
wherein the spacing member is configured to lower the bone flap from a raised position and secure the bone flap at the lowered position, the lowering movement being perpendicular to the first and second bone surfaces, and
wherein the external tool comprises a magnetic head (202) and wherein the spacing member comprises a magnetic portion (140), and wherein the magnetic head and the magnetic portion are magnetically coupleable transcutaneously, whereby rotation of the external tool causes a rotation of the spacing member for adjustment thereof,
**characterised in that** the spacing member is configured to lower the bone flap from a raised position upon receipt of the input from the external tool.

2. An adjustable bone implant according to claim 1, wherein in the lowered position, the first bone surface is aligned with the second bone surface and the first and second bone surfaces form a substantially contiguous surface.

3. An adjustable bone implant according to claim 1 or claim 2, wherein the body comprises a hollow interior and the spacing member is housed at least partially within the hollow interior.

4. An adjustable bone implant according to claim 3, wherein the body further comprises a sealed bottom.

5. An adjustable bone implant according to claim 3 or claim 4, wherein the spacing member comprises a first member with a screw thread, matingly engaged with a corresponding screw thread on an inside surface of the body, and whereby the input comprises rotation of the first member in a first direction, which causes an axial translation of the first member relative to the body to lower the bone flap from the raised position towards the lowered position, the axis of translation being perpendicular to the first and second bone surfaces.

6. An adjustable bone implant according to claim 3 or claim 4, wherein the spacing member comprises an inner member with a screw thread matingly engaged with a corresponding screw thread on an outer member, which in turn has a further screw thread matingly engaged with a corresponding screw thread on an inside surface of the body, and whereby the input comprises rotation of the inner member in a first direction, which causes an axial translation of the inner member relative to the outer member and in turn causes an axial translation of the outer member relative to the body to lower the bone flap from the raised position towards the lowered position, the axis of translation being perpendicular to the first and second bone surfaces.

7. An adjustable bone implant according to any preceding claim, wherein the magnetic portion is made from any of a magnetic alloy, aluminium alloy, stainless steel or neodymium.

8. An adjustable bone implant according to any preceding claim, wherein the magnetic head comprises one or more of either or both of: electromagnets or permanent magnets.

9. An adjustable bone implant according to any preceding claim, wherein any of the body or anchoring member is secured by at least two bone screws.

10. An adjustable bone implant according to any preceding claim, wherein the spacing member is extendable and retractable by up to 40 mm to respectively raise or lower the bone flap relative to the remainder of the skull by a corresponding amount.

11. An adjustable bone implant according to any preceding claim, wherein any of the spacing member, the anchoring member and the body are made from any of: carbon fibre, titanium, titanium alloy or stainless steel.

12. An adjustable bone implant according to any preceding claim, wherein the spacing member comprises a recess configured to receive a head of an external tool, and wherein the recess is accessible via a transcutaneous incision.

13. An adjustable bone implant according to any preceding claim, wherein the spacing member is connected to the anchoring member by means of an aperture through the anchoring member.

14. An adjustable bone implant according to any of claims 1 to 13, wherein the anchoring member comprises a hollow cylindrical shroud member, and wherein the spacing member is connected to the anchoring member by means of an underside of a top of the shroud member resting on top of the spacing member.

15. A kit of parts comprising:
an adjustable bone implant according to any preceding claim; and
said external tool comprising a magnetic head.

## Patentansprüche

1. Einstellbares Knochenimplantat (100) für die expandierbare Kranioplastik-Chirurgie, umfassend:
einen Körper (110), der konfiguriert ist, um an einer ersten Knochenoberfläche befestigt zu werden;
ein Verankerungselement (130), das konfiguriert ist, um an einer zweiten Knochenoberfläche befestigt zu werden, die in einer erhöhten Position versetzt von der ersten Knochenoberfläche gehalten wird, wobei die zweite Knochenoberfläche eine obere Oberfläche eines Knochendeckels (10) umfasst und die erste Knochenoberfläche eine obere Oberfläche des Rests des Schädels (20) umfasst; und
ein Abstandselement (120), das das Verankerungselement mit dem Körper auf einstellbare Weise verbindet, um das Verankerungselement selektiv vom Körper zu beabstanden,
wobei sich das Abstandselement im Gebrauch subkutan befindet,
wobei das Abstandselement konfiguriert ist, um eine Eingabe über eine transkutane Übertragung von einem externen Werkzeug (200) zu empfangen, und
wobei das Abstandselement konfiguriert ist, um den Knochendeckel aus einer angehobenen Position abzusenken und den Knochendeckel in der abgesenkten Position zu befestigen, wobei die Absenkbewegung senkrecht zu der ersten und zweiten Knochenoberfläche ist, und
wobei das externe Werkzeug einen Magnetkopf (202) umfasst und wobei das Abstandselement einen magnetischen Abschnitt (140) umfasst und wobei der Magnetkopf und der magnetische Abschnitt transkutan magnetisch koppelbar sind, wodurch eine Drehung des externen Werkzeugs eine Drehung des Abstandelements zur Einstellung
davon bewirkt,
**dadurch gekennzeichnet, dass** das Abstandselement konfiguriert ist, um den Knochendeckel aus einer angehobenen Position nach Empfang der Eingabe von dem externen Werkzeug abzusenken.

2. Einstellbares Knochenimplantat nach Anspruch 1, wobei in der abgesenkten Position die erste Knochenoberfläche mit der zweiten Knochenoberfläche ausgerichtet ist und die erste und zweite Knochenoberfläche eine im Wesentlichen zusammenhängende Oberfläche bilden.

3. Einstellbares Knochenimplantat nach Anspruch 1 oder Anspruch 2, wobei der Körper ein hohles Inneres umfasst und das Abstandselement zumindest teilweise in dem hohlen Inneren untergebracht ist.

4. Einstellbares Knochenimplantat nach Anspruch 3, wobei der Körper ferner einen abgedichteten Boden umfasst.

5. Einstellbares Knochenimplantat nach Anspruch 3 oder Anspruch 4, wobei das Abstandselement ein erstes Element mit einem Schraubengewinde umfasst, das passend mit einem entsprechenden Schraubengewinde an einer Innenfläche des Körpers in Eingriff steht, und wobei die Eingabe eine Drehung des ersten Elements in einer ersten Richtung umfasst, was eine axiale Translation des ersten Elements in Bezug auf den Körper bewirkt, um den Knochendeckel aus der angehobenen Position zur abgesenktem Position abzusenken, wobei die Translationsachse senkrecht zu der ersten und zweiten Knochenoberfläche ist.

6. Einstellbares Knochenimplantat nach Anspruch 3 oder Anspruch 4, wobei das Abstandselement ein Innenelement mit einem Schraubengewinde umfasst, das passend mit einem entsprechenden Schraubengewinde an einem Außenelement in Eingriff steht, das wiederum ein weiteres Schraubengewinde umfasst, das passend mit einem entsprechenden Schraubengewinde an einer Innenfläche des Köpers in Eingriff steht und wobei die Eingabe eine Drehung des Innenelements in einer ersten Richtung umfasst, was eine axiale Translation des Innenelements in Bezug auf das Außenelement bewirkt und wiederum eine axiale Translation des Außenelements in Bezug auf den Außenkörper bewirkt, um den Knochendeckel aus der angehobenen Position zur abgesenkten Position abzusenken, wobei die Translationsachse senkrecht zu der ersten und zweiten Knochenoberfläche ist.

7. Einstellbares Knochenimplantat nach einem der vorhergehenden Ansprüche, wobei der magnetische Abschnitt aus einem von einer magnetischen Legierung, Aluminiumlegierung, rostfreiem Stahl oder Neodym hergestellt ist.

8. Einstellbares Knochenimplantat nach einem der vorhergehenden Ansprüche, wobei der Magnetkopf einen oder mehrere von einem oder beiden umfasst von: Elektromagneten oder Permanentmagneten.

9. Einstellbares Knochenimplantat nach einem der vorhergehenden Ansprüche, wobei eines von dem Körper oder dem Verankerungselement durch mindestens zwei Knochenschrauben befestigt ist.

10. Einstellbares Knochenimplantat nach einem der vorhergehenden Ansprüche, wobei das Abstandselement um bis zu 40 mm ausfahrbar und einziehbar ist, um den Knochendeckel in Bezug auf den Rest des Schädels um einen entsprechenden Betrag anzuheben bzw. abzusenken.

11. Einstellbares Knochenimplantat nach einem der vorhergehenden Ansprüche, wobei eines von dem Abstandselement, dem Verankerungselement und dem Körper hergestellt sind aus einem von: Kohlefaser, Titan, Titanlegierung oder rostfreiem Stahl.

12. Einstellbares Knochenimplantat nach einem der vorhergehenden Ansprüche, wobei das Abstandselement eine Aussparung umfasst, die konfiguriert ist, um einen Kopf eines externen Werkzeugs aufzunehmen, und wobei die Aussparung über einen transkutanen Einschnitt zugänglich ist.

13. Einstellbares Knochenimplantat nach einem der vorhergehenden Ansprüche, wobei das Abstandselement mit dem Verankerungselement mittels einer Öffnung durch das Verankerungselement verbunden ist.

14. Einstellbares Knochenimplantat nach einem der Ansprüche 1 bis 13, wobei das Verankerungselement ein hohlzylindrisches Ummantelungselement umfasst und wobei das Abstandselement mit dem Verankerungselement mittels einer Unterseite einer Oberseite des Ummantelungselements verbunden ist, die auf der Oberseite des Abstandselements ruht.

15. Teilesatz, umfassend:
ein einstellbares Knochenimplantat nach einem der vorhergehenden Ansprüche; und
das externe Werkzeug, das einen Magnetkopf umfasst.

## Revendications

1. Implant osseux réglable (100) destiné à une chirurgie de cranioplastie expansive, comprenant :
un corps (110) conçu pour être fixé à une première surface osseuse ;
un élément d'ancrage (130) conçu pour être fixé à une seconde surface osseuse maintenue dans une position surélevée décalée par rapport à la première surface osseuse, ladite seconde surface osseuse comprenant une surface supérieure d'un volet osseux (10) et ladite première surface osseuse comprenant une surface supérieure du reste du crâne (20) ; et
un élément d'espacement (120) reliant l'élément d'ancrage au corps de manière réglable, pour espacer sélectivement l'élément d'ancrage du corps,
ledit élément d'espacement se trouvant sous la peau lors de l'utilisation,
ledit élément d'espacement étant conçu pour recevoir une entrée via une transmission transcutanée à partir d'un outil externe (200) et
ledit élément d'espacement étant conçu pour abaisser le volet osseux à partir d'une position surélevée et fixer le volet osseux en position abaissée, le mouvement d'abaissement étant perpendiculaire aux première et seconde surfaces osseuses, et
ledit outil externe comprenant une tête magnétique (202) et ledit élément d'espacement comprenant une partie magnétique (140), et ladite tête magnétique et ladite partie magnétique pouvant être couplées magnétiquement sous la peau, grâce à quoi la rotation de l'outil externe provoque une rotation de l'élément d'espacement pour le réglage de celui-ci,
**caractérisé en ce que** l'élément d'espacement est conçu pour abaisser le volet osseux à partir d'une position surélevée lors de la réception de l'entrée de l'outil externe.

2. Implant osseux réglable selon la revendication 1, dans la position abaissée, ladite première surface osseuse étant alignée avec la seconde surface osseuse et lesdites première et seconde surfaces osseuses formant une surface sensiblement contiguë.

3. Implant osseux réglable selon la revendication 1 ou la revendication 2, ledit corps comprenant un intérieur creux et ledit élément d'espacement étant logé au moins partiellement dans l'intérieur creux.

4. Implant osseux réglable selon la revendication 3, ledit corps comprenant en outre un fond scellé.

5. Implant osseux réglable selon la revendication 3 ou la revendication 4, ledit élément d'espacement comprenant un premier élément doté d'un filetage de vis, en prise par accouplement avec un filetage de vis correspondant sur une surface interne du corps, et grâce à quoi l'entrée comprend la rotation du premier élément dans un premier sens, qui provoque une translation axiale du premier élément par rapport au corps pour abaisser le volet osseux de la position surélevée vers la position abaissée, l'axe de translation étant perpendiculaire aux première et seconde surfaces osseuses.

6. Implant osseux réglable selon la revendication 3 ou la revendication 4, ledit élément d'espacement comprenant un élément interne doté d'un filetage de vis en prise par accouplement avec un filetage de vis correspondant sur un élément externe, qui à son tour possède un autre filetage de vis en prise par accouplement avec un filetage de vis correspondant sur une surface interne du corps, et grâce à quoi l'entrée comprend une rotation de l'élément interne dans un premier sens, ce qui provoque une translation axiale de l'élément interne par rapport à l'élément externe et provoque à son tour une translation axiale de l'élément externe par rapport au corps pour abaisser le volet osseux de la position surélevée vers la position abaissée, l'axe de translation étant perpendiculaire aux première et seconde surfaces osseuses.

7. Implant osseux réglable selon l'une quelconque des revendications précédentes, ladite partie magnétique étant constituée de l'un quelconque parmi un alliage magnétique, un alliage d'aluminium, un acier inoxydable ou le néodyme.

8. Implant osseux réglable selon l'une quelconque des revendications précédentes, ladite tête magnétique comprenant un ou plusieurs parmi les uns et/ou les autres : les électro-aimants ou les aimants permanents.

9. Implant osseux réglable selon l'une quelconque des revendications précédentes, l'un quelconque parmi le corps ou l'élément d'ancrage étant fixé par au moins deux vis à os.

10. Implant osseux réglable selon l'une quelconque des revendications précédentes, ledit élément d'espacement étant extensible et rétractable jusqu'à 40 mm pour élever ou abaisser respectivement le volet osseux par rapport au reste du crâne d'une quantité correspondante.

11. Implant osseux réglable selon l'une quelconque des revendications précédentes, l'un quelconque parmi l'élément d'espacement, l'élément d'ancrage et le corps étant constitué de l'un quelconque parmi : une fibre de carbone, le titane, un alliage de titane ou un acier inoxydable.

12. Implant osseux réglable selon l'une quelconque des revendications précédentes, ledit élément d'espacement comprenant un évidement conçu pour recevoir une tête d'un outil externe, et ledit évidement étant accessible via une incision transcutanée.

13. Implant osseux réglable selon l'une quelconque des revendications précédentes, ledit élément d'espacement étant relié à l'élément d'ancrage au moyen d'une ouverture à travers l'élément d'ancrage.

14. Implant osseux réglable selon l'une quelconque des revendications 1 à 13, ledit élément d'ancrage comprenant un élément d'enveloppe cylindrique creux et ledit élément d'espacement étant relié à l'élément d'ancrage au moyen d'une face inférieure d'une partie supérieure de l'élément d'enveloppe reposant sur le dessus de l'élément d'espacement.

15. Kit de pièces comprenant :
un implant osseux réglable selon l'une quelconque des revendications précédentes ; et
ledit outil externe comprenant une tête magnétique.
